# EUROPEAN PATENT SPECIFICATION

(11) **EP 1 926 472 B1**
(45) Date of publication and mention of the grant of the patent: **13.04.2011**
(21) Application number: 06802303.5
(22) Date of filing: 24.08.2006
(51) Int. Cl.: A61K 9/00, A61K 31/4535

(54) **STABILIZED AND PRESERVED KETOTIFEN OPHTHALMIC COMPOSITIONS**
STABILISIERTE UND KONSERVIERTE KETOTIFEN ENTHALTENDE OPHTHALMOLOGISCHE ZUSAMMENSETZUNGEN
COMPOSITIONS OPHTALMIQUES CONTENANT KETOTIFÈNE - STABILISÉES ET CONSERVÉES

(30) Priority: 26.08.2005 US 212957
(43) Date of publication of application: 04.06.2008
(73) Proprietor: Novartis AG, 4056 Basel (CH); Novartis Pharma GmbH, 1230 Wien (AT)
(72) Inventor: TSAO, Fu-Pao, Lawrenceville, GA 30043 (US)
(74) Representative: Barbier, Denis Robert
(86) International application number: PCT/US2006/033163
(87) International publication number: WO 2007/025094

(56) References cited:
- WO-A-00/19981
- WO-A-03/059069
- WO-A-2005/089715
- US-A1- 2002 127 281
- WALKER C V ET AL: "RHEOLOGICAL SYNERGISM BETWEEN IONIC CELLULOSE GUMS AND NONIONIC CELLULOSE GUMS" INTERNATIONAL JOURNAL OF PHARMACEUTICS, AMSTERDAM, NL, vol. 11, no. 4, 1982, pages 309-322, XP002331110 ISSN: 0378-5173

## Description

### Background of the Invention

The present invention relates to ophthalmic compositions comprising ketotifen as a pharmaceutically active agent, a source of hydrogen peroxide as a preservative and specific viscosity enhancers for use in treating allergic conjunctivitis. U.S. Patent Nos. 5,725,887 (the '887 patent) and 5,607,698, and Application Serial No. 11/078,209, disclose and claim methods for the preservation of ophthalmic solutions using stabilized hydrogen peroxide alone or together with the viscosity enhancing agents, hydroxypropyl methylcellulose (HPMC) and carboxymethylcellulose (CMC) and compositions so preserved. However, hydrogen peroxide is a strong oxidation agent Many chemical or drug substances are not compatible with hydrogen peroxide, i.e., are susceptible to chemical oxidation by hydrogen peroxide. Ketotifen is a pharmaceutically active agent susceptible to chemical oxidation. Stable ketotifen compositions (containing hydrogen peroxide as a preservative can be obtained by formulating the compositions at an acidic pH, e.g., at a pH of 3.5 to 6.0, as described in the application of Tsao, F., Wong, M. and Yen, S. filed on the same day as the present application. The application of the aforementioned stable ketotifer compositions to the eye particularly at high concentrations of ketotifen, e.g., 0.05% ketotifen, however, may cause stinging in the eye.

WO00109981 discloses an ophthalmic solution comprising chlorite/H₂O₂ (hydrogen peroxide) solutions sufficiently stably formulated in a combination with polymeric non-ionic and/or anionic lubricants.

In WO03059069 an aqueous ophthalmic solution is described which comprises by weight: 0.2% hydroxypropylmethylcellulose, 0.27% sodium chloride, 0.12% potassium chloride, 0.5% boric acid, 0.05% calcium chloride dihydrate, 0.006% diethylenetriamine penta (methylene phosphonic acid), and 0. 028% sodium perborate tetrahydrate, wherein the pH of said solution is between about 6.8 and about 7.0. One of the active agents which could be incorporated is ketotifen/ketotifen fumarate.

In US2002/0127281 a preserved aqueous ophthalmic solution comprises (a) a source of hydrogen peroxide - sufficient to provide hydrogen peroxide in an amount from about 2 ppm to 1000 ppm, namely hydrogen peroxide, sodium perborate, sodium peroxide or urea peroxide, (b) one or more hydrogen peroxide stabilizers (diethylene triamine penta(methylenephosphonic acid, 0.002-0.03%), (c) carboxymethylcellulose, (d) an aqueous solution with a pH of between about 8.0 and 10.5.

It has now been found that the eye comfort of these stable compositions formulated with high concentrations of ketotifen and HPMC can be improved by the addition of CMC. Further, it has been found that the bioavailability of the stable ketotifen compositions can be improved by the addition of HPMC alone or combined with CMC, thus providing stable, comfortable ketotifen compositions having significant clinical efficacy lasting for at least 16 hours.

### Summary of the Invention

In one aspect, an ophthalmic composition is provided which comprises:
(a) a ketotifen salt;
(b) a hydrogen peroxide source providing hydrogen peroxide in a trace amount of from 0.001 to 0.1% (w/v);
(c) one or more ocularly compatible hydrogen peroxide stabilizers;
(d) hydroxypropyl methylcellulose; and
(e) sodium carboxymethylcellulose, wherein the composition is at a pH sufficient to stabilize the ketotifen salt against oxidation by hydrogen peroxide.

In another aspect, a method for the treatment and prevention of allergic conjunctivitis is provided which comprises topically administering to a subjects suffering from or susceptible to the allergic conjunctivitis an effective amount of an ophthalmic composition comprising:
(a) a ketotifen salt;
(b) a hydrogen peroxide source providing hydrogen peroxide in a trace amount of from 0.001 to 0.1 % (w/v);
(c) one or more ocularly-compatible hydrogen peroxide stabilizers;
(d) hydroxypropyl methylcellulose; and
(e) carboxymethylcellulose, wherein the composition is at a pH sufficient to stabilize the ketotifen salt from oxidation by hydrogen peroxide.

### Detained Description of the Invention

The present invention is directed to stable ophthalmic compositions comprising a ketotifen salt, a hydrogen peroxide source providing hydrogen peroxide in a trace amount of from 0.001 to 0.1 % (w/v), one or more ocularly-compatible hydrogen peroxide stabilizers, HPMC and CMC. The compositions are formulated at a pH sufficient to stabilize the ketotifen salt against oxidation by hydrogen peroxide e.g., at a pH of from 3.5 to 6.0. Thus, the acid form of ketotifen is more stable than the neutral form of ketotifen. Inclusion of CMC to HPMC-containing ketotifen compositions formulated at a pH that stabilizes the ketotifen against oxidation by hydrogen peroxide improves the comfort of the stablized compositions upon application to the eye. Stablized ketotifen compositions containing both HPMC and CMC also posses a partition coefficient that is about three fold greater than ketotifen/hydrogen peroxide compositions containing glycerol (see Examples 1 and 3), indicating that the addition of HPMC and CMC to these composition, may also enhance drug penetration and increase drug bioavailability. That the inclusion of HPMC and CMC to the stabilized ketotifen compositions may enhance drug penetration and increase drug bioavailability is further supported by results from clinical studies described herein which demonstrate that these compositions are efficacious in treating and preventing allergic conjunctivitis and possess a long duration of action with clinically significant itch reduction persisting to 16 hours after dosing (See Example 4). In view of its clinical efficacy for at least 16 hours, the ophthalmic compositions of the present invention can be administered once-per-day.

The ketotifen salt is, e.g., ketotifen hydrochloride, ketotifen hydrobromide, ketotifen pamoate, ketotifen maleate, ketotifen sulfate and ketotifen fumarate. A preferred ketotifen salt is ketotifen fumarate. The concentration of ketotifen salt as ketotifen is typically from 0.01 to 0.1 % (weight/volume, w/v) and is preferably from 0.02 to 0.06% (wlv).

Trace amounts of peroxy compounds stabilized with a hydrogen peroxide stabilizer, especially diethylene triamine penta(methylene phosphonic acid) or 1-hydroxyethylidene-1,1-diphosphonic acid may be utilized as a preservative for the ophthalmic compositions of the invention. A hydrogen peroxide source is any peroxy compound that is hydrolyzed in water to produce hydrogen peroxide. Examples of hydrogen peroxide sources which provide an effective resultant amount of hydrogen peroxide, include sodium perborate, sodium perborate tetrahydrate, sodium peroxide and urea peroxide. It has been found that peracetic acid, an organic peroxy compound, cannot be stabilized utilizing the present system.

The hydrogen peroxide source is present in an amount sufficient to result in from 0.001 to 0.1% (w/v) hydrogen peroxide, and preferably from 0.001 to 0.01% (w/v). As an example, the hydrogen peroxide source, sodium perborate tetrahydrate, can be present in an amount of from 0.005 to 0.5%(w/v).

A "hydrogen peroxide stabilizer", as used herein, means any of the known stabilizers of peroxy compounds including phosphonates, phosphates, stannates, etc. Physiologically compatible salts of phosphonic acids may also be used, such as diethylene triamine penta(methylene-phosphonic acid) and physiologically compatible salts thereof and 1-hydroxyethylene-1,1,-diphosphonic acid and physiological acceptable salts thereof. Other stabilizers of peroxy compounds useful in the practice of the present invention are disclosed in the '887 patent at, *inter alia,* column 5, line 55 to column 6, line 34.

The above stabilizers can be used in almost all indications previously mentioned to which the invention is applicable. However, when the solution is to come in contact with a hydrogel soft contact lens, stannate stabilizers are to be avoided as they tend to "cloud" the lens material.

When the peroxy stabilizer is diethylene triamine penta(methylene-phosphonic acid, it can be present in the ophthalmic compositions in an amount between 0.001 to 0.02% (w/v), or in an amount between 0.002 and 0.012% (w/v).

When the peroxy stabilizer is 1-hydroxyethylene-1,1,-diphosphonic acid it can be present in the ophthalmic compositions in an amount between 0.002 and 0.2% (w/v) of the composition.

Stabilizers other than diethylene triamine penta(methylene-phosphonic acid) (sold by Monsanto Company, St. Louis, Mo., under the trademark DEQUEST 2060) and physiologically compatible salts thereof and 1-hydroxyethylene-1,1,-diphosphonic acid and physiologically acceptable salts thereof are employed in physiologically tolerable amounts.

Soluble alkaline earth metal salts can be used in the compositions in amounts between 0.002 and 0.2% (w/v) of the composition, or between 0.01% and 0.1% by weight of the composition. Water-soluble salts of magnesium and calcium are such alkaline earth metal salts. Addition of such soluble alkaline earth metal salts increases antifungal preservative efficacy in ophthalmic compositions preserved with low amounts of hydrogen peroxide.

The pH of the stabilized ophthalmic composition of the present invention is adjusted to a pH value sufficient to stabilize the ketotifen salt against oxidation by hydrogen peroxide, e.g., a pH of from 3.5 to 6.0. Preferably, the pH of the ophthalmic composition is from 3.8 to 5.5, and more preferably from 4.0 to 5.3. The pH can be adjusted as desired by incorporation of suitable amounts of acid or base of a physiologically tolerable nature in the amounts employed, e.g., hydrochloric acid and sodium hydroxide.

There may be present in the ophthalmic compositions according to the present invention one or more conventional, substantially inert, physiologically acceptable tonicity enhancing agents. Suitable such agents include e.g., mannitol, sorbitol, glycerol, alkali metal halides, phosphates, hydrogen phosphate and borates. Preferred are sodium chloride, sodium phosphate monobasic and sodium phosphate dibasic. The function of such tonicity enhancing agents is to assure approximate physiologic tonicity to the composition which is instilled in the eye or to help assure such tonicity upon dilution if dilution is necessary prior to contact with the eye cue to peroxide content as indicated above.

Preferably sufficient tonicity enhancing agents are present in the solution so that it is substantially isotonic or, such that, upon decomposition or dilution of the hydrogen peroxide therein, the resulting composition is substantially isotonic, e.g., substantially equivalent in tonicity to a 0.9% by weight aqueous sodium chloride solution. Preferably, the amount of tonicity enhancing agent present in the ophthalmic composition is from 0,01 to 1 % (w/v).

The ophthalmic compositions of the present invention further comprise both viscosity enhancing agents, HPMC and CMC. Suitable grades of HPMC are Methocel A, E, F, J, and K brand products from Dow Chemical and suitable grades of CMC are Akucell Al= 2781, Aqualon 7H3SXF PH, 7L, 7M from Akzo Nobel, Aqualon. The HPMC concentration in the compositions of the invention is from about 0.005 to about 1%(wlv) and is preferably from about 0.1 to about 0.5% (w/v). In one embodiment, the HPMC concentration is from 0.1 to 0.5%(w/v). The CMC concentration in the compositions of the invention is from 0.005 to 0.5% (w/v) and is preferably from 0.05 to 0.4%(w/v). In a particularly useful embodiment, the HPMC and CMC concentrations in a composition of the present invention are 0.3% and 0.10%, respectively.

In one embodiment, the ophthalmic composition of the present invention comprises:
(a) 0.0138 to 0.138%(wlv) of ketotifen fumarate;
(b) 0.005 to 0.5%(w/v) of sodium perborate,
(c) 0.001 to 0.02%(w/v) of diethylenetriamine penta(methylene phosphonic acid) and physiological salts thereof or 0.002 to 0.2% *(w*/*v)* of 1-hydroxyethylidene-1-diphosphonic acid and physiological salts thereof;
(d) 0.005 to 1%(w/v) of HPMC; and
(e) 0.005 to 0.5%(w/v) of CMC, wherein the composition is at a pH of from 3.5 to 6.0.

In a particularly useful embodiment, the ophthalmic composition of the present invention comprises:
(a) 0.069% (w/v) of ketotifen fumarate;
(b) 0.028 %(w/v) of sodium perborate tetrahydrate;
(c) 0.006% (w/v) of diethylenetriamine penta(methylene phosphonic acid);
(d) 0.30% (w/v) of HPMC;
(e) 0.10% (w/v) of CMC; and
(f) 0.64% (w/v) of NaCl, wherein the pH of the composition is from 4.0 to 5.3. Purified water is added to bring the total to 100%.

The ophthalmic compositions of the present invention are also characterized by their extraordinary stability, even under accelerated conditions, e.g., by heating the solutions to 100°C for 24 hours. Thus, the shelf life of these compositions is enhanced. Moreover, the instant compositions are characterized by physiological tolerability subsequent to hydrogen peroxide decomposition.

Another advantage in using hydrogen peroxide in ophthalmic compositions is that the trace amount of hydrogen peroxide, especially less than 100 ppm, is destroyed once comes in contact with the eye. For example, catalase existing in the eye tissue will cause the breakdown of the hydrogen peroxide into water and oxygen. As a result, the composition, upon application, becomes preservative free and greatly minimizes adverse reactions. The problems associated with other preservatives, such as the inability to break down innocuous compounds, are eliminated.

Compositions of the invention can be made in any conventional manner. For example, all of the components other than the hydrogen peroxide and water can be placed in a container and fresh, preferably concentrated, hydrogen peroxide added thereto with mixing. Alternatively, the dry components can be rubbed up with a small portion of liquid stabilizer, then the remainder of the stabilizer added, followed by the hydrogen peroxide, and most of the water. Other components, e.g., tonicity adjusting agents can then be added or the formed composition can be added to such agents.

In one embodiment of preparing the composition, HPMC is added to a container containing water, heated and mixed at a temperature of 80 to 90°C until dispersed followed by the addition of CMC and mixed until dissolved. The solution containing the two polymers is then sterilized at a temperature of at least 121°C. The final sterile solution is cooled to room temperature and mixed until dissolved. The ketotifen fumarate and sodium chloride are added to a separate container and mixed until dissolved. The solution of ketotifen fumarate and sodium chloride is then sterile filtered into the container containing the HPMC/CMC solution and mixed until dissolved. The mixture is then brought up to final volume with sterile filtered water and the pH is adjusted by the addition of hydrochloric acid or sodium hydroxide. One of ordinary skill in the art will be aware of numerous variations in the manner of formulating the compositions of the invention.

Another aspect of the present invention is directed to an effective amount of the aforementioned ophthalmic composition for use to be topically administered to a subject suffering from or susceptible to the allergic conjunctivitis for treating and preventing allergic conjunctivitis.

In one embodiment the ophthalmic composition can be applied directly to the eye, preferably in the form of eye drops for the treatment and reduction in itching of the eye due to allergic conjunctivitis and for the treatment and reduction of signs or any symptoms of seasonal allergic conjunctivitis. Dosage of the ophthalmic composition will depend on severity of the allergic conjunctivitis and the concentration of the ketotifen salt in the composition and can be readily determined by one skilled in the art. Typically, between one and ten, or between one and five, or between one and three drops are administered at one time when employing the compositions of the present invention. As an example, at high concentrations of a ketotifen salt such as 0.069% (w/v) ketotifen fumarate, one drop can be applied topically once-per-day.

In yet another embodiment of this method, the ophthalmic compositions can also be formulated for use in contact lens care solutions, e.g., contact lens wetting, storing, lubricating, cleaning, disinfecting solutions and cosmetic care solutions.

The ophthalmic compositions of the present invention can be packaged in any pharmaceutically acceptable packaging, but it is desirable to package them in squeezable plastic multidose containers, such as dropper bottles, Such bottles can be made, e.g., of polyethylene or polypropylene or mixtures thereof. A dropper bottle will typically dispense between about 25 mL and about 50mL per drop. When it is desirable to "neutralize" the peroxide activity, any means known, such as rinsing, contacting the solution with platinum, catalase or any other substance known to decompose hydrogen peroxide, will suffice. Additional physiological compatible peroxide neutralizing agents include reducing agent, such as pyruvic acid; and suitable salts thereof, such as the sodium salt.

The following examples are presented for illustrative purposes and are not intended to limit the scope of this invention, but to demonstrate the stability of the ophthalmic compositions as stabilized in accordance with the present invention. All parts are by %(w/v) unless otherwise indicated.

### EXAMPLE 1

Formulations 1-7 (Tables 1-7) are prepared containing the indicated ingredients. Each formulation is filled in a masked label drop bottle and the two formulations are given to 6 to 7 patients by instilling one drop of each formulation into each eye. The comfort level of Formulation 1 (containing HPMC and CMC, and Formulation 7 (containing HPMC alone) is evaluated following instillation in a human eye and graded from severe stinging, mild stinging, uncomfortable, reasonable comfort, to comfort. The partition coefficient of n-octanol versus aqueous solution for Formulations 2-7 is measured by pipetting an equal amount of octanol and aqueous solution into a glass bottle with a cap. The bottle with solution is then vigorously shaken for 30 minutes and then left to stand still until there is a complete separation of two layers. The aqueous layer is then pipetted and the concentration of ketotifen (A) is measured to compare with the concentration of ketotifen before the bottle was shook (B). The partition coefficient (D) is calculated as follows: D=(B-A)/A.

**Table 1-Formulation 1**

| | |
|---|---|
| Ketotifen Fumarate | 0.069% (w/v) |
| HPMC | 0.3% (w/v) |
| Dequest 2060 | 0.006% (w/v) |
| Sodium Perborate | 0.028% (w/v) |
| CMC (sodium carboxymethylcellulose) | 0.1 % (w/v) |
| *Added* Purified Water to the volume | |
| *Adjusted* pH | 4.147 |
| Tonicity | 225 mOsm/kg |

**Table 2-Formulation 2**

| | |
|---|---|
| Ketotifen Fumarate | 0.069% |
| Glycerol | 2% |
| Dequest 2060 | 60 ppm |
| Sodium Perborate | 0.028% (w/v) |
| pH | 4.100 |
| Tonicity | 186 mOsm/kg |
| Partition Coefficient | 0.235 |

**Table 3-Formulation 3**

| | |
|---|---|
| Ketotifen Fumarate | 0.069% (w/v) |
| HPMC | 0.2% (w/v) |
| Dequest 2060 | 0.006% (w/v) |
| Sodium Perborate | 0.028% (w/v) |
| Sodium Chloride | 0.5% (w/v) |
| pH | 4.100 |
| Tonicity | 214 mOsm/kg |
| Partition Coefficient | 1.044 |

**Table 4-Formulations 4**

| | |
|---|---|
| Ketotifen Fumarate | 0.069% (w/v) |
| HPMC | 0.2% (w/v) |
| CMC | 0.1 % (w/v) |
| Dequest 2060 | 0.006% (w/v) |
| Sodium Perborate | 0.028% (w/v) |
| Sodium Chloride | 0.62% (w/v) |
| pH | 4.042 |
| Tonicity | 223 mOsm/kg |
| Partition Coefficient | 1.019 |

**Table 5-Formulation 5**

| | |
|---|---|
| Ketotifen Fumerate | 0.069% (w/v) |
| CMC | 0.1 % (w/v) |
| Dequest 2060 | 0.006% (w/v) |
| Sodium Perborate | 0.028% (w/v) |
| Sodium Chloride | 0.6% (w/v) |
| pH | 4.034 |
| Tonicity | 207 mOsm/kg |
| Partition Coefficient | 1.053 |

**Table 6-Formulation 6**

| | |
|---|---|
| Ketotifen Fumarate | 0.069% (w/v) |
| CMC | 0.1 % (w/v) |
| Calcium Chloride | 0.05% (w/v) |
| Dehydrate | |
| Magnesium Chloride | 0.05% (w/v) |
| Hexahydrate | |
| Dequest 2060 | 0.006% (w/v) |
| Sodium Perborate | 0.028% (w/v) |
| Sodium Chloride | 0.5% (w/v) |
| pH | 4.027 (w/v) |
| Tonicity | 193 mOsm/kg |
| Partition Coefficient | 1.014 |

**Table 7-Formulation 7**

| | |
|---|---|
| Ketotifen Fumarate | 0.069% (w/v) |
| HPMC | 0.3% (w/v) |
| Boric Acid | 0.5% (w/v) |
| Potassium Chloride | 0.12% (w/v) |
| Dequest 2060 | 0.006% (w/v) |
| Sodium Perborate | 0.028% (w/v) |
| Sodium Chloride | 0.31% (w/v) |
| pH | 4.058 |
| Tonicity | 212 mOsm/kg |

### EXAMPLE 2

The results of the study in Example 1 evaluating the comfort level of Formulations 1 and 7 are shown below in Table 8.

**Table 8-Results of Comfort Study**

| **Formulation** | **Feeling on Eye** |
|---|---|
| 7 | Severely stinging and uncomfortable |
| 1 | Reasonable comfort |

The results in Table 8 demonstrate that Formulation 1 containing both HPMC and CMC possesses a higher comfort level than Formulation 7 containing HPMC alone following instillation into the eye.

### EXAMPLE 3

The results of the study in Example 1 evaluating the partition coefficient of Formulations 2-6 are summarized in Table 9 below.

**Table 9-Results of Partition Coefficient Study**

| **Formulation** | **Partition Coefficient of *n*-Octanol vs. Aqueous Solution** |
|---|---|
| 2 | 0.235 |
| 3 | 1.044 |
| 4 | 1.019 |
| 5 | 1.053 |
| 6 | 1.014 |

The results in Table 9 demonstrate that the partition coefficient is significantly enhanced in formulations containing both CMC and HPMC.

### EXAMPLE 4 A Randomized, Double Masked, Placebo-Controlled Evaluation of the Onset and Duration of Action of Reformulated Zaditor (Ketotifen Fumarate 0.05%) Compared with Vehicle in the Conjunctival Allergen Challenge (CAC) Model

This study compares the efficacy of a single dose of ketotifen fumarate 0.05 % ophthalmic solution [Formula: 0.069% (w/v) of Ketotifen fumarate, 0.30 % (w/v) of HPMC, 0.10% (w/v) of CMC, 0.028 % (w/v) of sodium perborate tetrahydrate, 0.006% (w/v) of phosphonic acid-Dequest 2060S, 0.64% (w/v) sodium chloride and water, pH from 4.0 to 5.0] versus matched vehicle-placebo [Formula: 0.30 % (w/v) of HPMC, 0.10% (w/v) of CMC, 0.028 % (w/v) of sodium perborate tetrahydrate, 0.006% (w/v) of phosphonic acid-Dequest 2060S, 0.64% (w/v) sodium chloride and water, pH from 4.0 to 5.0) in subjects with a history of allergic conjunctivitis to cat hair, cat dander, ragweed and/or trees 15 minutes and 16 hours after instillation using the CAC model. The objective is to determine if 0.069% (w/v) ketotifen fumarate (equivalent to 0.05% free base) has a 15-minute onset of action and 16-hour duration of action.

### A. Methodology

This is a single-center, randomized, double-masked, placebo-controlled, parallel-group, allergen challenge study involving 4 study visits: Visit 1 [Day 0, screening Conjunctival Provocation Test (CPT)], Visit 2 (Day 7 ± 2, CAC), Visit 3 (Day 21 ± 3, randomization, 16-hour post-dose allergen challenge), and Visit 4 (Day 35 ± 3, 15-minute post-dose challenge, study exit). At Visit 1, a CPT is conducted to establish the subject's response to 1 or more ocular allergens. After an allergen concentration is found to produce a successful response, reproducibility of the response is validated by a confirmatory allergen challenge at Visit 2. At that time, eligible subjects are randomly assigned (ratio of 1:2:2:1) to 1 of 4 treatment groups: ketotifen fumarate 0.05% OU; ketotifen fumarate 0.05% OD, placebo OS; ketotifen fumarate 0.05% OS, placebo OD; or placebo OU. Each of the groups receive a 1-drop dose of an ophthalmic solution in each eye at Visits 3 and 4. Both Visits 3 and 4 are scheduled after a 2-week washout period. An allergen challenge is conducted 16 hours post-dose at Visit 3 and 15 minutes post-dose at Visit 4.

Approximately 100 subjects are planned for screening to provide 60 complete subjects without any major protocol violations. A total of 54 subjects are randomized and receive study drug at Visits 3 and 4 (9, ketotifen OU; 9, placebo OU; 36, ketotifen/placebo). All subjects are evaluated for efficacy and safety.

### B. Criteria for Evaluation

The primary efficacy variable is ocular itching (0-4 scale), which is evaluated at 3, 5, and 7 minutes after the 16-hour and 15-minute post-dose allergen challenges at Visits 3 and 4, respectively. Secondary efficacy variables include composite ocular hyperemia (conjunctival, ciliary and episcleral) (same 0-4 scale, each vessel bed); chemosis (0-4 scale); mucous discharge (absent or present); tearing (absent or present); lid swelling (0-3 scale); and a composite nasal symptom score. Secondary ocular variables are evaluated at 5, 7, and 15 minutes after the 16-hour and 15-minute allergen challenges. Nasal symptoms are assessed at 10, 20 and 30 minutes post-challenge. With each scale, lower scores are indicative of better status (milder intensity).

### C. Statistical Methods

Efficacy analyses are conducted on the "intent-to-treat" (ITT) population, defined as all randomized subjects who receive at least 1 dose of study medication and have post-baseline efficacy data. No methods are used to impute for missing data. Safety analyses include all available data from subjects who receive at least 1 dose of study medication and have post-baseline safety data. No interim analyses are planned or conducted. All statistical tests are 2-sided, and tests with a corresponding P value ≤0.050 are statistically significant.

In the primary analysis of ocular itching, ketotifen fumarate 0.05% ophthalmic solution is compared for superiority over placebo. Descriptive statistics are used to summarize mean and mean changes from baseline (defined as the post-challenge scores at Visit 2) in ocular itching scores. Eyes from both the bilaterally- and contralaterally-treated groups are included in the analysis according to treatment. Least square means are calculated and analyzed using an analysis of variance (ANOVA) model containing a factor for treatment. Mean differences between treatments and their associated two-sided 95% confidence intervals (Cls) are reported. Clinically significant between-treatment differences are defined as differences of at least 1 unit. The percentage of subjects who report an itching score equal to zero (responder analysis) are compared between treatments using Fisher's exact test. All analyses are completed for each post-dose and post-challenge time. Methods analogous to those utilized for the primary efficacy variable are applied to all other efficacy variables. For binary variables, such as tearing and mucous discharge, only percentages and Fisher's exact test results are reported. Nasal endpoints are treated as coming from 3 treatment levels - active treatment in both eyes, active treatment in a single eye and placebo in both eyes. As a secondary analysis, differences in ocular itching, hyperemia, lid swelling and chemosis are analyzed using paired t-tests in the portion of subjects who receive ketotifen fumarate 0.05% in 1 eye and placebo in the other eye.

### D. Results-Efficacy

At each evaluation and timepoint, eyes treated with ketotifen 0.05% exhibit significantly (P <0.001) less itching after allergen challenge compared with placebo-treated eyes (Table 10). At 16 hours post-dose (Visit 3), the mean difference between treatments in the ocular itching score is clinically-significant (defined as a difference of >1 unit) at the 5-minute post-CAC evaluation (-1.05 units) and approaches clinical significance at the 7-minute timepoint (-0.96 units). For the allergen challenge conducted at 15 minutes post-dose (Visit 4), clinically significant differences are observed between treatments at 3, 5 and 7 minutes post-challenge (-1.52, -1.65 and -1.51 units, respectively).

**Table 10. Primary Efficacy - Ocular Itching After Dosing (ITT)**

| **Assessment Time Post-challenge** | **Treatment** | **Mean Score** | **Between-treatment Difference and 95% CI (Active - Placebo)¹** | **P Value²** |
|---|---|---|---|---|
| **15 minutes post-dose** | | | | |
| 3 minutes | Ketotifen 0.05% | 0.40 | -1.52 (-1.87, -1.17) | <0.001 |
| | Placebo | 1.93 | | |
| 5 minutes | Ketotifen 0.05% | 0.40 | -1.65 (-1.98, -1.33) | <0.001 |
| | Placebo | 2.06 | | |
| 7 minutes 0.05% | Ketotifen | 0.46 | -1.51 (-1.83,-1.19) | <0.001 |
| | Placebo | 1.97 | | |
| **16 hours post-dose** | | | | |
| 3 minutes | Ketotifen 0.05% | 0.94 | -0.87 (-1.25,-0.49) | <0.001 |
| | Placebo | 1.81 | | |
| 5 minutes | Ketotifen 0.05% | 0.96 | -1.05 (-1.41, -0.69) | <0.001 |
| | Placebo | 2.01 | | |
| 7 minutes | Ketotifen 0.05% | 0.98 | -0.96 (-1.29, -0.64) | <0.001 |
| | Placebo | 1.94 | | |

| | | | | |
|---|---|---|---|---|
| ¹A negative difference indicates that active formulation has a better effect than placebo. ²Based on ANOVA. | | | | |

Similar trends are observed for the fellow-eye analysis of ocular itching (Table 11). Eyes treated with ketotifen 0.05% exhibits significantly (P <0.001) less itching after allergen challenge compared with placebo-treated eyes. Clinically significant differences are observed (in favor of ketotifen 0.05%) at each timepoint.

**Table 11. Primary Efficacy (Fellow Eye Analysis) - Ocular Itching After Dosing (ITT)**

| **Assessment Time Post-challenge** | **Treatment** | **Within-Treatment Score** | **Mean Between-treatment Difference (Active - Placebo)** | **P Value¹** |
|---|---|---|---|---|
| **15 minutes post-dose** | | | | |
| 3 minutes | Ketotifen 0.05% | 0.47 | -1.14 | <0.001 |
| | Placebo | 1.61 | | |
| 5 minutes | Ketotifen 0.05% | 0.42 | -1.38 | <0.001 |
| | Placebo | 1.79 | | |
| 7 minutes | Ketotifen 0.05% | 0.49 | -1.28 | <0.001 |
| | Placebo | 1.76 | | |
| **16 hours post-dose** | | | | |
| 3 minutes | Ketotifen 0.05% | 0.72 | -1.04 | <0.001 |
| | Placebo | 1.76 | | |
| 5 minutes | Ketotifen 0.05% | 0.78 | -1.17 | <0.001 |
| | Placebo | 1.94 | | |
| 7 minutes | Ketotifen 0.05% | 0.79 | -1.03 | <0.001 |
| | Placebo | 1.82 | | |

| | | | | |
|---|---|---|---|---|
| ¹Based on paired t-tests. | | | | |

At each evaluation and timepoint, eyes treated with ketotifen 0.05% have significantly (P <0.001) higher responder rates (defined as eyes with a score of 0 itching post-CAC) compared with placebo-treated eyes (Table 12). Approximately 35-40% of ketotifen-treated eyes have no itching after the allergen challenge conducted at 16 hours post-dose (Visit 3) versus 4-7% of placebo-treated eyes. This difference is more marked following the allergen challenge conducted 15 minutes after dosing at Visit 4.

**Table 12. Responder Analysis - No Ocular Itching After Dosing (ITT)**

| **Assessment Time Post-challenge** | **Treatment** | **% Responders** | | | |
|---|---|---|---|---|---|
| | | **15 minutes post-dose** | | **16 hours post-dose** | |
| | | No Itching¹ | P Value² | No Itching | P Value |
| 3 minutes | Ketotifen 0.05% | 63.5% | <0.001 | 40.7% | <0.001 |
| | Placebo | 7.4% | | 7.4% | |
| 5 minutes | Ketotifen 0.05% | 65.4% | <0.001 | 38.9% | <0.001 |
| | Placebo | 3.7% | | 5.6% | |
| 7 minutes | Ketotifen 0.05% | 63.5% | <0.001 | 35.2% | <0.001 |
| | Placebo | 3.7% | | 3.7% | |

| | | | | | |
|---|---|---|---|---|---|
| ¹Defined as an itching score equal to 0 units. ²P value based on Fisher's exact test. | | | | | |

Based on composite ocular hyperemia scores, eyes treated with ketotifen 0.05% exhibits less redness than placebo-treated eyes at each evaluation and timepoint (Table 13). Statistically significant differences are observed between treatments at 16 hours post-dose at 5 and 7 minutes post-CAC and at 15 minutes post-dose at 5, 7 and 15 minutes post-CAC. No clinically significant differences are observed between treatments (defined as a difference of ≥3 units). Similar trends are observed for each of the redness scores that contribute to the composite score (conjunctival, ciliary and episcleral). Results of the fellow-eye analyses of ocular hyperemia are comparable to those presented in Table 13.

**Table 13. Secondary Efficacy - Composite Ocular Hyperemia After Dosing (ITT)**

| **Assessment Time Post-challenge** | **Treatment** | **Mean Score** | **Between-treatment Difference and 95% CI (Active - Placebo)¹** | **P Value²** |
|---|---|---|---|---|
| **15 minutes post-dose** | | | | |
| 5 minutes | Ketotifen 0.05% | 2.14 | -1.78 (-2.70, -0.86) | <0.001 |
| | Placebo | 3.93 | | |
| 7 minutes | Ketotifen 0.05% | 2.81 | -1.68 (-2.62, -0.75) | <0.001 |
| | Placebo | 4.49 | | |
| 15 minutes | Ketotifen 0.05% | 3.94 | -1.12 (-2.11,-0.13) | 0.027 |
| | Placebo | 5.06 | | |
| **16 hours post-dose** | | | | |
| 5 minutes | Ketotifen 0.05% | 2.70 | -0.78 (-1.55, -0.01) | 0.047 |
| | Placebo | 3.48 | | |
| 7 minutes | Ketotifen 0.05% | 3.42 | -0.78 (-1.50, -0.05) | 0.036 |
| | Placebo | 4.19 | | |
| 15 minutes | Ketotifen 0.05% | 4.15 | -0.60 (-1.35, 0.14) | 0.112 |
| | Placebo | 4.75 | | |

| | | | | |
|---|---|---|---|---|
| ¹A negative difference indicates that active formulation has a better effect than placebo. ²Based on ANOVA. | | | | |

At each evaluation and timepoint, eyes treated with ketotifen 0.05% exhibited less lid swelling and chemosis after allergen challenge compared to placebo-treated eyes (Table 14). For lid swelling, these differences are not statistically significant at Visit 3 (16-hour post-dose challenge) but are significant at each post-CAC timepoint at Visit 4 (15-minute post-dose challenge). For chemosis, the differences are statistically significant at Visit 3 at 5 minutes post-CAC and at Visit 4 at 5, 7 and 15 minutes post-CAC. For both parameters, no clinically significant differences are observed between treatments (defined as a difference of ≥1 unit). Results of the fellow-eye analyses of lid swelling and chemosis are comparable to those presented in Table 14, although with the fellow-eye analysis, statistically significant differences (in favor of ketotifen 0.05%) are observed for both parameters at each timepoint.

**Table 14. Secondary Efficacy - Lid Swelling and Chemosis After Dosing (ITT)**

| **Assessment Time Post-challenge** | **Treatment** | **Mean Score** | **Between-treatment Difference and 95% Cl (Active - Placebo)¹** | **P Value²** |
|---|---|---|---|---|
| **Lid Swelling** | | | | |
| **15 minutes post-dose** | | | | |
| 5 minutes | Ketotifen 0.05% | 0.10 | -0.33 (-0.54, -0.12) | 0.002 |
| | Placebo | 0.43 | | |
| 7 minutes | Ketotifen 0.05% | 0.13 | -0.44 (-0.67, -0.21) | <0.001 |
| | Placebo | 0.57 | | |
| 15 minutes | Ketotifen 0.05% | 0.13 | -0.38 (-0.61, -0.16) | 0.001 |
| | Placebo | 0.52 | | |
| **16 hours post-dose** | | | | |
| 5 minutes | Ketotifen 0.05% | 0.13 | -0.19 (-0.38, 0.01) | 0.063 |
| | Placebo | 0.31 | | |
| 7 minutes | Ketotifen 0.05% | 0.22 | -0.19 (-0.40, 0.03) | 0.085 |
| | Placebo | 0.41 | | |
| 15 minutes | Ketotifen 0.05% | 0.37 | -0.11 (-0.36, 0.14) | 0.383 |
| | Placebo | 0.48 | | |
| **Chemosis** | | | | |
| **15 minutes post-dose** | | | | |
| 5 minutes | Ketotifen 0.05% | 0.23 | -0.18 (-0.33, -0.03) | 0.021 |
| | Placebo | 0.41 | | |
| 7 minutes | Ketotifen 0.05% | 0.32 | -0.23 (-0.38, -0.08) | 0.003 |
| | Placebo | 0.55 | | |
| 15 minutes | Ketotifen 0.05% | 0.41 | -0.18 (-0.35, -0.01) | 0.037 |
| | Placebo | 0.59 | | |
| **16 hours post-dose** | | | | |
| 5 minutes | Ketotifen 0.05% | 0.15 | -0.12 (-0.23, -0.01) | 0.031 |
| | Placebo | 0.27 | | |
| 7 minutes | Ketotifen 0.05% | 0.31 | -0.07 (-0.19, 0.04) | 0.189 |
| | Placebo | 0.39 | | |
| 15 minutes | Ketotifen 0.05% | 0.43 | -0.11 (-0.28, 0.05) | 0.183 |
| | Placebo | 0.54 | | |

| | | | | |
|---|---|---|---|---|
| ¹A negative difference indicates that active formulation has a better effect than placebo. ²Based on ANOVA. | | | | |

At each evaluation and timepoint, the percentage of subjects who exhibited tearing after allergen challenge are higher in placebo-treated eyes compared with ketotifen-treated eyes (Table 15). These differences are not statistically significant at any post-CAC timepoint at Visit 3 (16-hour post-dose challenge) but are significant at each timepoint at Visit 4 (15-minute post-dose challenge). None of the subjects have mucous discharge at any time after challenge at Visits 3 and 4.

**Table 15. Secondary Analysis - Tearing After Dosing (ITT)**

| **Assessment Time Post-challenge** | **Treatment** | **% of Subjects** | | | |
|---|---|---|---|---|---|
| | | **15 minutes post-dose** | | **16 hours post-dose** | |
| | | Tearing¹ | P Value² | Tearing | P Value |
| 5 minutes | Ketotifen 0.05% | 11.5% | 0.006 | 18.5% | 0.362 |
| | Placebo | 35.2% | | 27.8% | |
| 7 minutes | Ketotifen 0.05% | 9.6% | 0.001 | 20.4% | 0.132 |
| | Placebo | 37.8% | | 35.2% | |
| 15 minutes | Ketotifen 0.05% | 7.7% | 0.033 | 16.7% | 0.628 |
| | Placebo | 24.1% | | 22.2% | |

| | | | | | |
|---|---|---|---|---|---|
| ¹Defined as the presence of tearing. ²P value based on Fisher's exact test. | | | | | |

At each evaluation and timepoint, subjects in the bilateral ketotifen 0.05% group have a lower mean composite nasal symptom score after allergen challenge compared with subjects who receive placebo OU. However, due to the small number of subjects in each of these groups (N=9), caution should be exercised in interpretation of the results.

### E. Conclusions

From both a statistical and clinical perspective, the results of this allergen challenge study demonstrate that ketotifen fumarate 0.05% ophthalmic solution is significantly better than placebo for reducing the severity of ocular itching in susceptible subjects following allergen exposure. Ketotifen 0.05% demonstrates a rapid onset of effect (15 minutes) and long duration of action, with clinically significant itch reduction persisting to 16 hours after dosing. Ketotifen 0.05% also reduces other signs and symptoms associated with allergen exposure, namely ocular hyperemia, lid swelling, chemosis and tearing.

## Claims

1. An ophthalmic composition comprising:
(a) a ketotifen salt;
(b) a hydrogen peroxide source providing hydrogen peroxide in a trace amount of from 0.001 to 0.1 % (w/v);
(c) one or more ocularly compatible hydrogen peroxide stabilizers;
(d) hydroxypropyl methylcellulose; and
(e) sodium carboxymethylcellulose, wherein the composition is at a pH sufficient to stabilize the ketotifen salt against oxidation by hydrogen peroxide.

2. The composition of Claim 1, wherein the pH of the composition is from 3.5 to 6.0.

3. The composition of Claim 2, wherein the pH of the composition is from 4.0 to 5.3.

4. The composition of Claim 1, wherein the ketotifen salt is ketotifen fumarate at a concentration of from 0.01 to 0.1% (w/v).

5. The composition of Claim 1, wherein the hydrogen peroxide source is selected from the group consisting of sodium perborate, sodium perborate tetrahydrate, sodium peroxide and urea peroxide.

6. The composition of Claim 1, wherein the hydrogen peroxide source is from 0.001 and 0.01% (w/v).

7. The composition of Claim 1, wherein the one or more hydrogen peroxide stabilizers is selected from the group consisting of diethylene triamine penta(methylene phosphonic acid), 1-hydroxyethylidene-1,1-diphosphonic acid and physiologically compatible salts thereof.

8. The composition of Claim 7, wherein the composition comprises from 0.001 to 0.02% (w/v) of diethylene triamine penta(methylene phosphonic acid) or a physiologically compatible salt thereof.

9. The composition of Claim 7, wherein the composition comprises from 0.002 to 0.2% (w/v)1-hydroxyethylidene-1,1-diphosphonic acid or a physiologically compatible salt thereof.

10. The composition of Claim 1, wherein the composition further comprises a tonicity enhancing agent.

11. The composition of claim 1, wherein the concentration of hydroxypropyl methylcellulose is from 0.005 to 1% (w/v) and wherein the concentration of sodium carboxymethylcellulose is from 0.005 to 0.5% (w/v).

12. The composition of Claim 11, wherein the concentration of hydroxypropyl methylcellulose is from 0.1 to 0.5% (w/v) and wherein the concentration of sodium carboxymethylcellulose is from 0.04 to 0.4% (w/v).

13. An ophthalmic composition comprising:
a) 0.069% (w/v) of ketotifen fumarate;
b) 0.028% (w/v) of sodium perborate tetrahydrate;
c) 0.006% (w/v) of diethylenetriamine penta(methylene phosphonic acid),
d) 0.3% (w/v) of HPMC; and
e) 0.1% (w/v) of CMC, wherein the composition is at a pH of from 4.0 to 5.3.

14. An effective amount of an ophthalmic composition which comprises:
(a) a ketotifen salt;
(b) a hydrogen peroxide source providing hydrogen peroxide in a trace amount of from 0.001 to a 0.1% (w/v);
(c) one or more ocularly-compatible hydrogen peroxide stabilizers;
(d) hydroxypropyl methylcellulose; and
(e) carboxymethylcellulose, wherein the composition is at a pH sufficient to stabilize the ketotifen salt from oxidation by hydrogen peroxide
for use to be topically administered to a subject suffering from or susceptible to the allergic conjunctivitis for treating and preventing allergic conjunctivitis.

15. The ophthalmic composition of Claim 14, wherein the composition is at a pH of from 4.0 to 5.3.

16. The ophthalmic composition of Claim 14, wherein the ketotifen salt is ketotifen fumarate at a concentration of from 0.01 to 0.2% (w/v).

17. The ophthalmic composition of Claim 14, wherein the hydrogen peroxide source is selected from the group consisting of hydrogen peroxide, sodium perborate, sodium perborate tetrahydrate, sodium peroxide and urea peroxide.

18. The ophthalmic composition of Claim 14, wherein the concentration of the hydrogen peroxide source is from 0.001 to 0.01 % (w/v).

19. The ophthalmic composition of Claim 14, wherein the one or more hydrogen peroxide stabilizers is selected from the group consisting of diethylene triamine penta(methylene phosphonic acid), 1-hydroxyethylidene-1,1-diphosphonic acid and physiologically compatible salts thereof.

20. The ophthalmic composition of Claim 19, wherein the composition comprises from 0.001 to 0.02% (w/v) of diethylene triamine penta(methylene phosphonic acid) or a physiologically compatible salt thereof.

21. The ophthalmic composition of Claim 19, wherein the composition comprises from 0.002 to 0.2% (w/v) of 1-hydroxyethylidene-1,1-diphosphonic acid or a physiologically compatible salt thereof.

22. The ophthalmic composition of Claim 14, wherein the composition further comprises a tonicity adjusting agent selected from the group consisting of mannitol, sorbitol, glycerol, alkali metal halides, phosphates, hydrogen phosphate and borates in am amount from 0.01 to 1% (w/v).

23. The ophthalmic composition of Claim 14, wherein the ophthalmic composition comprises:
a) 0.069% (w/v) of ketotifen fumarate;
b) 0.028% (w/v) of sodium perborate tetrahydrate;
c) 0.006% (w/v) of diethylenetriamine penta(methylene phosphonic acid),
d) 0.30% (w/v) of HPMC; and
e) 0.10% (w/v) of CMC, wherein the composition is at a pH of from 4.0 to 5.3.

24. The ophthalmic composition of Claim 23, wherein the ophthalmic composition is administered once a day.

25. The ophthalmic composition of Claim 1 for use in preventing and treating allergic conjunctivitis.

## Patentansprüche

1. Ophthalmische Zusammensetzung, die folgenden Bestandteile umfasst:
(a) ein Ketotifensalz;
(b) eine Wasserstoffperoxidquelle, die Wasserstoffperoxid in einer Spurenmenge von 0,001 bis 0,1 % (g/v) liefert;
(c) einen oder mehrere okular verträgliche Wasserstoffperoxidstabilisatoren;
(d) Hydroxypropylmethylcellulose; und
(e) Natriumcarboxymethylcellulose, wobei die Zusammensetzung bei einem pH-Wert vorliegt, der ausreicht, um das Ketotifensalz gegen eine Oxidation durch Wasserstoffperoxid zu stabilisieren.

2. Zusammensetzung nach Anspruch 1, wobei der pH-Wert der Zusammensetzung in einem Bereich von 3,5 bis 6,0 liegt.

3. Zusammensetzung nach Anspruch 2, wobei der pH-Wert der Zusammensetzung in einem Bereich von 4,0 bis 5,3 liegt.

4. Zusammensetzung nach Anspruch 1, wobei das Ketotifensalz Ketotifenfumarat in einer Konzentration von 0,01 bis 0,1 % (g/v) ist.

5. Zusammensetzung nach Anspruch 1, wobei die Wasserstoffperoxidquelle aus der Gruppe ausgewählt ist, die aus Natriumperborat, Natriumperborattetrahydrat, Natriumperoxid und Harnstoffperoxid besteht.

6. Zusammensetzung nach Anspruch 1, wobei die Wasserstoffperoxidquelle in einem Bereich von 0,001 bis 0,01 % (g/v) vorhanden ist.

7. Zusammensetzung nach Anspruch 1, wobei ein oder mehrere Wasserstoffperoxidstabilisatoren aus der Gruppe ausgewählt sind, die aus Diethylentriaminpenta(methylenphosphonsäure), 1-Hydroxyethyliden-1,1-diphosphonsäure und physiologisch verträglichen Salzen hiervon besteht.

8. Zusammensetzung nach Anspruch 7, wobei die Zusammensetzung 0,001 bis 0,02 % (g/v) Diethylentriaminpenta(methylenphosphonsäure) oder ein physiologisch verträgliches Salz hiervon umfasst.

9. Zusammensetzung nach Anspruch 7, wobei die Zusammensetzung 0,002 bis 0,2 % (g/v) 1-Hydroxyethyliden-1,1-diphosphonsäure oder ein physiologisch verträgliches Salz hiervon umfasst.

10. Zusammensetzung nach Anspruch 1, wobei die Zusammensetzung ferner ein die Tonizität erhöhendes Mittel umfasst.

11. Zusammensetzung nach Anspruch 1, wobei die Konzentration von Hydroxypropylmethylcellulose 0,005 bis 1 % (g/v) beträgt und die Konzentration von Natriumcarboxymethylcellulose 0,005 bis 0,5 % (g/v) beträgt.

12. Zusammensetzung nach Anspruch 11, wobei die Konzentration von Hydroxypropylmethylcellulose 0,1 bis 0,5 % (g/v) beträgt und die Konzentration von Natriumcarboxymethylcellulose 0,04 bis 0,4 % (g/v) beträgt.

13. Ophthalmische Zusammensetzung, die die folgenden Bestandteile umfasst:
(a) 0,069 % (g/v) Ketotifenfumarat;
(b) 0,028 % (g/v) Natriumperborattetrahydrat;
(c) 0,006 % (g/v) Diethylentriaminpenta(methylenphosphonsäure);
(d) 0,3 % (g/v) HPMC; und
(e) 0,1 % (g/v) CMC, wobei die Zusammensetzung bei einem pH-Wert von 4,0 bis 5,3 vorliegt.

14. Wirksame Menge einer ophthalmischen Zusammensetzung, die die folgenden Bestandteile umfasst:
(a) ein Ketotifensalz;
(b) eine Wasserstoffperoxidquelle, die Wasserstoffperoxid in einer Spurenmenge von 0,001 bis 0,1 % (g/v) liefert;
(c) einen oder mehrere okular verträgliche Wasserstoffperoxidstabilisatoren;
(d) Hydroxypropylmethylcellulose; und
(e) Carboxymethylcellulose, wobei die Zusammensetzung bei einem pH-Wert vorliegt, der ausreicht, um das Ketotifensalz vor einer Oxidation durch Wasserstoffperoxid zu stabilisieren,
wobei die wirksame Menge der ophthalmischen Zusammensetzung topisch an einen Patienten, der an allergischer Konjunktivitis leidet oder dafür empfänglich ist, zur Behandlung oder Verhinderung von allergischer Konjunktivitis verabreicht wird.

15. Ophthalmische Zusammensetzung nach Anspruch 14, wobei die Zusammensetzung bei einem pH-Wert von 4,0 bis 5,3 vorliegt.

16. Ophthalmische Zusammensetzung nach Anspruch 14, wobei das Ketotifensalz Ketotifenfumarat in einer Konzentration von 0,01 bis 0,2 % (g/v) ist.

17. Ophthalmische Zusammensetzung nach Anspruch 14, wobei die Wasserstoffperoxidquelle aus der Gruppe ausgewählt ist, die aus Wasserstoffperoxid, Natriumperborat, Natriumperborattetrahydrat, Natriumperoxid und Harnstoffperoxid besteht.

18. Ophthalmische Zusammensetzung nach Anspruch 14, wobei die Konzentration der Wasserstoffperoxidquelle 0,001 bis 0,01 % (g/v) beträgt.

19. Ophthalmische Zusammensetzung nach Anspruch 14, wobei der eine oder die mehreren Wasserstoffperoxidstabilisatoren aus der Gruppe ausgewählt sind, die aus Diethylentriaminpenta(methylenphosphonsäure), 1-Hydroxyethyliden-1,1-diphosphonsäure und physiologisch verträglichen Salzen hiervon besteht.

20. Ophthalmische Zusammensetzung nach Anspruch 19, wobei die Zusammensetzung 0,001 bis 0,02 % (g/v) Diethylentriaminpenta(methylenphosphonsäure) oder ein physiologisch verträgliches Salz hiervon umfasst.

21. Ophthalmische Zusammensetzung nach Anspruch 19, wobei die Zusammensetzung 0,002 bis 0,2 % (g/v) 1-Hydroxyethyliden-1,1-diphosphonsäure oder ein physiologisch verträgliches Salz hiervon umfasst.

22. Ophthalmische Zusammensetzung nach Anspruch 14, wobei die Zusammensetzung ferner ein die Tonizität einstellendes Mittel, das aus der Gruppe ausgewählt ist, die aus Mannit, Sorbit, Glycerin, Alkalimetallhalogeniden, Phosphaten, Hydrogenphosphat und Boraten besteht, in einer Menge von 0,01 bis 1 % (g/v), umfasst.

23. Ophthalmische Zusammensetzung nach Anspruch 14, wobei die ophthalmische Zusammensetzung die folgenden Bestandteile umfasst:
(a) 0,069 % (g/v) Ketotifenfumarat;
(b) 0,028 % (g/v) Natriumperborattetrahydrat;
(c) 0,006 % (g/v) Diethylentriaminpenta(methylenphosphonsäure);
(d) 0,30 % (g/v) HPMC; und
(e) 0,10 % (g/v) CMC, wobei die Zusammensetzung bei einem pH-Wert von 4,0 bis 5,3 vorliegt.

24. Ophthalmische Zusammensetzung nach Anspruch 23, wobei die ophthalmische Zusammensetzung einmal täglich verabreicht wird.

25. Ophthalmische Zusammensetzung nach Anspruch 1 zur Verhinderung und Behandlung von allergischer Konjunktivitis.

## Revendications

1. Composition ophtalmique comprenant :
(a) un sel de kétotifène ;
(b) une source de peroxyde d'hydrogène fournissant du peroxyde d'hydrogène en une quantité infime allant de 0,001 à 0,1 % (p/v) ;
(c) un ou plusieurs stabilisants du peroxyde d'hydrogène présentant une compatibilité oculaire ;
(d) de l'hydroxypropylméthylcellulose ; et
(e) de la carboxyméthylcellulose de sodium, ladite composition étant à un pH suffisant pour stabiliser le sel de kétotifène contre une oxydation par le peroxyde d'hydrogène.

2. Composition selon la revendication 1, dans laquelle le pH de la composition va de 3,5 à 6,0.

3. Composition selon la revendication 2, dans laquelle le pH de la composition va de 4,0 à 5,3.

4. Composition selon la revendication 1, dans laquelle le sel de kétotifène est le fumarate de kétotifène à une concentration allant de 0,01 à 0,1 % (p/v).

5. Composition selon la revendication 1, dans laquelle la source de peroxyde d'hydrogène est choisie dans le groupe constitué par le perborate de sodium, le perborate de sodium tétrahydraté, le peroxyde de sodium et le peroxyde d'urée.

6. Composition selon la revendication 1, dans laquelle la source de peroxyde d'hydrogène va de 0,001 à 0,01 % (p/v).

7. Composition selon la revendication 1, dans laquelle le ou les stabilisants du peroxyde d'hydrogène sont choisis dans le groupe constitué par la diéthylènetriamine penta(acide méthylène phosphonique), l'acide 1-hydroxyéthylidène-1,1-diphosphonique et leurs sels physiologiquement compatibles.

8. Composition selon la revendication 7, ladite composition comprenant 0,001 à 0,02 % (p/v) de diéthylènetriamine penta(acide méthylène phosphonique) ou de l'un de ses sels physiologiquement compatibles.

9. Composition selon la revendication 7, ladite composition comprenant 0,002 à 0,2 % (p/v) d'acide 1-hydroxyéthylidène-1,1-diphosphonique ou de l'un de ses sels physiologiquement compatibles.

10. Composition selon la revendication 1, ladite composition comprenant en outre un agent améliorant la tonicité.

11. Composition selon la revendication 1, dans laquelle la concentration d'hydroxypropylméthylcellulose va de 0,005 à 1 % (p/v) et dans laquelle la concentration de carboxyméthylcellulose de sodium va de 0,005 à 0,5 % (p/v).

12. Composition selon la revendication 11, dans laquelle la concentration d'hydroxypropylméthylcellulose va de 0,1 à 0,5 % (p/v) et dans laquelle la concentration de carboxyméthylcellulose de sodium va de 0,04 à 0,4 % (p/v).

13. Composition ophtalmique comprenant :
a) 0,069 % (p/v) de fumarate de kétotifène ;
b) 0,028 % (p/v) de perborate de sodium tétrahydraté ;
c) 0,006 % (p/v) de diéthylènetriamine penta(acide méthylène phosphonique) ;
d) 0,3 % (p/v) de HPMC ; et
e) 0,1 % (p/v) de CMC, ladite composition étant à un pH de 4,0 à 5,3.

14. Quantité efficace d'une composition ophtalmique comprenant :
(a) un sel de kétotifène ;
(b) une source de peroxyde d'hydrogène fournissant du peroxyde d'hydrogène en une quantité infime allant de 0,001 à 0,1 % (p/v) ;
(c) un ou plusieurs stabilisants du peroxyde d'hydrogène présentant une compatibilité oculaire ;
(d) de l'hydroxypropylméthylcellulose ; et
(e) de la carboxyméthylcellulose, ladite composition étant à un pH suffisant pour stabiliser le sel de kétotifène contre une oxydation par le peroxyde d'hydrogène, et qui est administrée localement à un sujet souffrant de ou susceptible de souffrir d'une conjonctivite allergique, afin de traiter et de prévenir la conjonctivite allergique.

15. Composition ophtalmique selon la revendication 14, ladite composition étant à un pH de 4,0 à 5,3.

16. Composition ophtalmique selon la revendication 14, dans laquelle le sel de kétotifène est le fumarate de kétotifène à une concentration allant de 0,01 à 0,2 % (p/v).

17. Composition ophtalmique selon la revendication 14, dans laquelle la source de peroxyde d'hydrogène est choisie dans le groupe constitué par le peroxyde d'hydrogène, le perborate de sodium, le perborate de sodium tétrahydraté, le peroxyde de sodium et le peroxyde d'urée.

18. Composition ophtalmique selon la revendication 14, dans laquelle la concentration de la source de peroxyde d'hydrogène va de 0,001 à 0,01 % (p/v).

19. Composition ophtalmique selon la revendication 14, dans laquelle le ou les stabilisants du peroxyde d'hydrogène sont choisis dans le groupe constitué par la diéthylènetriamine penta(acide méthylène phosphonique), l'acide 1-hydroxyéthylidène-1,1-diphosphonique et leurs sels physiologiquement compatibles.

20. Composition ophtalmique selon la revendication 19, ladite composition comprenant 0,001 à 0,02 % (p/v) de diéthylènetriamine penta(acide méthylène phosphonique) ou de l'un de ses sels physiologiquement compatibles.

21. Composition ophtalmique selon la revendication 19, ladite composition comprenant 0,002 à 0,2 % (p/v) d'acide 1-hydroxyéthylidène-1,1-diphosphonique ou de l'un de ses sels physiologiquement compatibles.

22. Composition ophtalmique selon la revendication 14, ladite composition comprenant en outre un agent d'ajustement de la tonicité choisi dans le groupe constitué par le mannitol, le sorbitol, le glycérol, les halogénures de métaux alcalins, les phosphates, le phosphate d'hydrogène et les borates en une quantité de 0,01 à 1 % (p/v).

23. Composition ophtalmique selon la revendication 14, ladite composition ophtalmique comprenant :
a) 0,069 % (p/v) de fumarate de kétotifène ;
b) 0,028 % (p/v) de perborate de sodium tétrahydraté ;
c) 0,006 % (p/v) de diéthylènetriamine penta(acide méthylène phosphonique) ;
d) 0,30 % (p/v) de HPMC ; et
e) 0,10 % (p/v) de CMC, ladite composition étant à un pH allant de 4,0 à 5,3.

24. Composition ophtalmique selon la revendication 23, ladite composition ophtalmique étant administrée une fois par jour.

25. Composition ophtalmique selon la revendication 1 pour la prévention et le traitement de la conjonctivite allergique.
